# EUROPEAN PATENT APPLICATION

(11) **EP 3 338 829 A1**
(43) Date of publication of application: **27.06.2018**
(21) Application number: 16206619.5
(22) Date of filing: 23.12.2016
(51) Int. Cl.: A61M 5/00

(54) **MEDICAL DEVICE PACKAGING**

(71) Applicant: Sanofi-Aventis Deutschland GmbH, 65929 Frankfurt (DE)
(72) Inventor: The designation of the inventor has not yet been filed

(57) **Abstract**

A medical device packaging comprising: a container having an opening; a medical device disposed within the container, the medical device including an energy storage apparatus; a first layer comprising a main part disposed at least partially over a peripheral edge of the opening of the container; and a second layer disposed over the main part of the layer, wherein the first layer is in electrical contact with the energy storage apparatus and the separation of the lining layer from the conductive layer generates electrostatic energy for charging the energy storage apparatus.

## Description

### Field

The present invention relates to a medical device packaging.

### Background of the Invention

A variety of diseases exist that require regular treatment by injection of a medicament and such injections can be performed by using injection devices. Injection devices known in the art include infusion and patch pumps for delivering injections of medicament. Another type of injection device is a bolus injector device. Some bolus injector devices are intended to be used with relatively large volumes of medicament, typically at least 1 ml and maybe a few ml. Injection of such large volumes of medicament can take some minutes or even hours. Such high capacity bolus injector devices can be called large volume devices (LVDs). Generally such devices are operated by the patients themselves, although they may also be operated by medical personnel.

To use an injector device, such as an LVD, it is first supported on a suitable injection site on a patient's skin. Once installed, injection is initiated by the patient or another person (user). Typically, the initiation is effected by the user operating an electrical switch, which causes a controller to operate the device. Operation includes firstly injecting a needle into the user and then causing the injection of medicament into the user's tissue. Biological medicaments are being increasingly developed which comprise higher viscosity injectable liquids and which are to be administered in larger volumes than long-known liquid medicaments. LVDs for administering such biological medicaments may comprise a pre-filled disposable drug delivery device or, alternatively, a disposable drug delivery device into which a patient or medical personnel must insert a drug cartridge prior to use.

The drug delivery process using such injection devices may include numerous steps, including removing one or more device components from their respective packaging, assembly and/or preparing of the components to ready the device for medicament administration and attachment of the injection device to a suitable injection site on the body, before the actual process of injecting the medicament can begin. Such processes can therefore be complicated and, particularly in the case of patient-operated devices, may be difficult for the patient to determine the status of the device.

In addition, in some patient-operated LVDs, the drug delivery process from start to finish may be a lengthy process and sometimes it is difficult for the patient to determine whether the injection process is complete. Some medicament delivery devices are provided with on-board equipment including light sources and indicator systems for indicating the amount of medicament currently contained within the device. In some of these devices with on-board equipment, batteries are provided so as to power the on-board equipment. Battery life is therefore an important consideration for patient-operated LVDs.

### Summary of the Invention

According to an aspect of the present invention, there is provided a medical device packaging comprising a container having an opening; a medical device disposed within the container, the medical device including an energy storage apparatus; a first layer comprising a main part disposed at least partially over a peripheral edge of the opening of the container; and a second layer disposed over the main part of the layer, wherein the first layer is in electrical contact with the energy storage apparatus and the separation of the lining layer from the conductive layer generates electrostatic energy for charging the energy storage apparatus.

The main part of the first layer may be at least partially adhesive.

The medical device packaging may further comprise an electrical contact apparatus, wherein electrostatic energy generated at the first layer is transferred through the electrical contact apparatus to the energy storage apparatus.

The electrical contact apparatus may comprise electrical contact pads arranged to allow electrical connection of the first layer to the energy storage apparatus when the medical device is disposed within the container.

The first layer may comprise an extension part which is arranged to extend towards the electrical contact apparatus such that the first layer is in electrical contact with the electrical contact apparatus.

The energy storage apparatus may comprise a capacitor.

The medical device may further comprise a light source arranged to be powered by energy stored in the energy storage apparatus

The light source may comprise at least one light-emitting diode.

The medical device may be a bolus injector.

The medical device may contain liquid medicament.

The container may be deformable.

According to another aspect of the present invention, there is provided a method for preparing a medical device packaging for use, the medical device packaging comprising a container having an opening; a medical device disposed within the container, the medical device including an energy storage apparatus; a first layer disposed at least partially over a peripheral edge of the opening of the container, the first layer being in electrical contact with the energy storage apparatus; and a second layer disposed over the first layer, the method comprising separating the second layer from the first layer and thereby causing generation of electrostatic energy for charging the energy storage apparatus.

These and other aspects of the invention will be apparent from and elucidated with reference to the embodiments described hereinafter.

### Brief Description of the Drawings

Exemplary embodiments of the present invention are described with reference to the accompanying drawings, in which:
Figure 1 shows a perspective view of a medical device packaging of an embodiment of the present invention, in a first partially opened state; and
Figure 2 shows a perspective view of the medical device packaging of Figure 1 in a second partially opened state.

Reference will now be made in detail to the embodiments, examples of which are illustrated in the accompanying drawings, wherein like reference numerals refer to like elements throughout.

### Details Description of the Embodiments

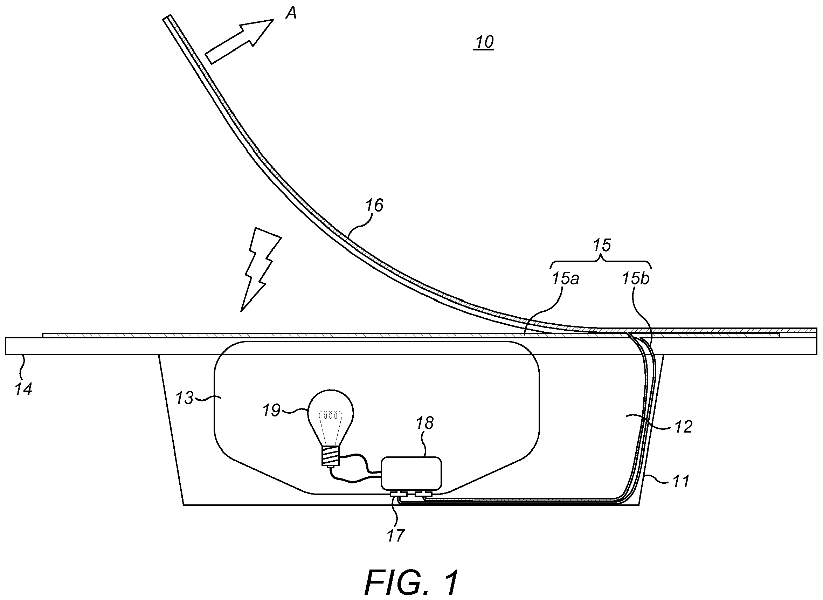

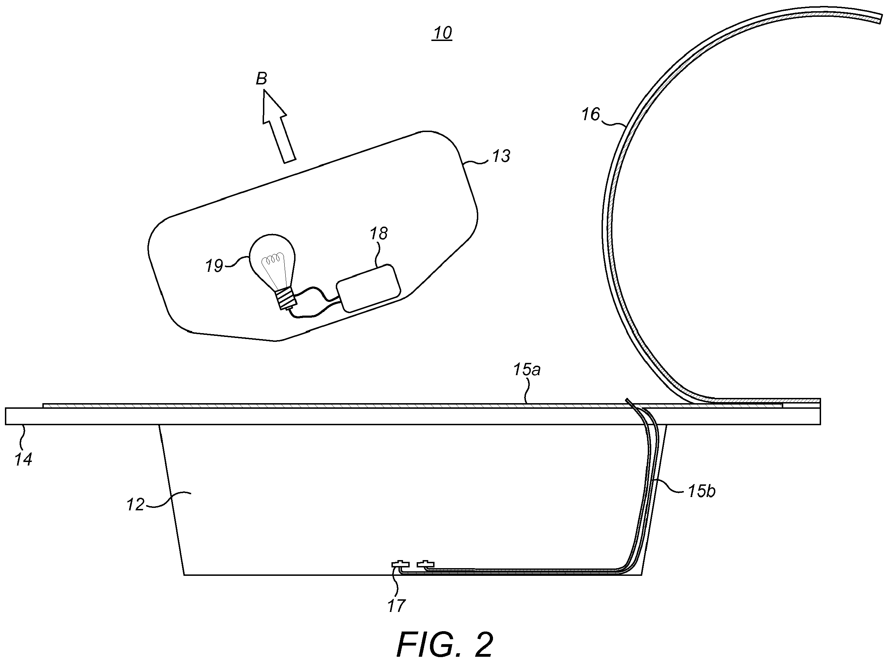

Figures 1 and 2 show a medical device packaging 10 according to an embodiment of the present disclosure, respectively in a first partially opened state and a second partially opened state. The medical device packaging 10 comprises a packaging container 11 (hereafter "container 11") having a recess defining an interior space 12. A medical device 13 is received within the interior space 12. The medical device 13 (hereafter "device 13") may be a medicament delivery device such as a medicament injection deice, for example an infusion pump, patch pump, a bolus injector device or an LVD.

As shown in Figure 1, the device 13 comprises an energy storage apparatus 18 and a light source 19. In an initial state, such as what is shown in Figure 1, i.e. when the device 13 is contained inside the interior space 12 of the container 11, the energy storage apparatus 18 is in electrical connection with an electrical contact apparatus 17. In the present embodiment, the electrical contact apparatus 17 is positioned at a bottom of the interior space 12 of the container 11. Also, in the present embodiment, the electrical contact apparatus 17 comprises electrical contact pads that are arranged to allow electrical connection between the energy storage apparatus 18 of the device 13 and an extension part 15b of a first layer 15. The first layer 15 will be explained in further detail below.

In the present embodiment, the energy storage apparatus 18 comprises a capacitor. The energy storage apparatus 18 is electrically connected to the light source 19, which in the present embodiment comprises a light-emitting diode (LED). As described above, when the device 13 is placed inside the interior space 12 and on top of the electrical contact apparatus 17, electrical connection between the energy storage apparatus 18 of the device 13 and the extension part 15b of the first layer 15 is established.

The container 11 may be formed of a flexible or a deformable material. Such material may, for example, comprise plastic, foil or card. This may enable a user to deform the container 11 to push the device 13 out of the container once the device 13 is secure to an injection site. The container 11 further comprises a flange 14 extending radially outwards around a peripheral edge of the opening of the interior space 12. Part of the first layer 15 is applied over the flange 14 and is bonded to the flange 14. Specifically, a main part 15a of the first layer 15 is applied over the flange 14.

The first layer 15 is an electrically conductive layer, and is therefore made of electrically conductive material, e.g. conductive polymer. The first layer 15 comprises the main part 15a and the extension part 15b. As described above, the main part 15a of the first layer 15 is applied around the opening of the recess which defines the interior space 12 of the container 11, and is adhesive such that it is releasably attached to a second layer 16 in an initial state. The extension part 15b of the first layer 15 is arranged to extend towards the electrical contact apparatus 17 such that it is in electrical contact with the electrical contact apparatus 17. Therefore, while the device 13 is contained within the container 11 and placed on top of the electrical contact apparatus 17, an electrical connection can be established between the first layer 15 and the energy storage apparatus 18 of the device 13.

The second layer 16 is applied over the first layer 15 initially so as to cover the opening of the recess which defines the interior space 12 of the container 11. The second layer 16 may be referred to as a lining layer. Since the second layer 16 initially covers the opening of the recess which defines the interior space 12, the medical device 13 can be securely sealed within the interior space 12 of the container 11. As described above, the second layer 16 is releasably attached to the main part 15a of the first layer 15. When the second layer 16 is separated from the main part 15a of the first layer 15, electrostatic energy is generated between the main part 15a of the first layer 15 and the second layer 16 due to triboelectric effect.

The generated electrostatic energy generated between the main part 15a of the first layer 15 is conducted through the extension part 15b towards the electrical contact apparatus 17 positioned at the bottom of the interior space 12. As such, when the device 13 is properly positioned within the interior space 12 such that the energy storage apparatus 18 is in electrical connection with the electrical contact apparatus 17, the generated electrostatic energy can be stored in the energy storage apparatus 18, i.e. charging the energy storage apparatus 18 with the generated electrostatic energy. The stored energy in the energy storage apparatus 18 can then be used for powering the light source 19 or other components (not shown in the drawing) of the device 13. For example, the stored energy in the energy storage apparatus 18 may be used to power the light source 19 so as to indicate an end of an injection process. The device 13 may be programmed so as to achieve this particular effect or other desired effects.

A sequence of operation of the medical device packaging 10 according to the present embodiment is as follows:
The medical device packaging 10 is initially in a sealed state where the second layer fully covers the recess which defines the interior space 12 of the container 11. To access the device 13, a user grabs hold of a tab (not shown in the drawings) of the second layer 16 and peels the second layer 16 in a direction as indicated by arrow A in Figure 1. This peeling action causes the separation of the second layer 16 from the main part 15a of the first layer 15, thereby generating electrostatic energy by means of contact electrification. The generated electrostatic energy is conducted through the main part 15a of the first layer 15, through the extension part 15b of the first layer, and to the energy storage apparatus 18 of the medical device 13 via the electrical contact apparatus 17. The generated electrostatic energy is therefore stored in the energy storage apparatus 18.

The user then places the medical device 13 onto an injection site and pushes the device 13 out of the container 11 in the direction as indicated by arrow B in Figure 2. The device 13 can then be used to administer medicament in accordance with the manufacturer's instructions. For example, the user may trigger an injection to the injection site by means of pushing an actuation button provided at the device 13. When the injection is complete, the light source 19 of the medical device 13 is powered on by the stored energy in the energy storage apparatus 18 so as to indicate to the user that the injection is complete.

Although it is described above that the container includes a flange extending outwardly from the edge of the container, the invention is not limited to this configuration and the container may not necessarily include such a defined flange, and may alternatively include an edge or rim portion around the opening of the container. The main part of the first layer may therefore be bonded to the rim or the edge of the container opening. Such rim or edge may include a flat portion to facilitate the main part of the first layer bonding to the container. In embodiments having a flange, it will be appreciated that the flange provides such a flat portion for bonding of the main part of the first layer to the container.

Although it is described above that the main part of the first layer is applied over the flange, in alternative embodiments the main part of the first layer may be disposed only partially over a peripheral edge of the opening of the container of the medical device packaging.

Although it is described above that the energy storage apparatus is in electrical connection with the first layer via the electrical contact apparatus, in alternative embodiments the electrical contact apparatus may not be used for establishing electrical connection between the energy storage apparatus and the first layer. In these alternative embodiments, the first layer may be arranged to be directly electrically connected to the energy storage apparatus in the initial state.

Although it is described above that the main part of the first layer is adhesive, in alternative embodiments the main part of the first layer may be only partially adhesive.

In alternative embodiments, the electrical contact apparatus may not be located at a bottom of the interior space of the container. For example, in some alternative embodiments, the electrical contact apparatus may be arranged on a side of the interior space of the container. In these alternative embodiments, the energy storage apparatus of the medical device may be arranged to correspond to the position of the electrical contact apparatus.

In alternative embodiments, the energy storage apparatus may comprise other types of energy storage devices instead of a capacitor.

In alternative embodiments, the light source may comprise other types of lighting devices instead of an LED.

In alternative embodiments, instead of a light source, other types of indicating apparatuses may be provided. For example, the medical device may comprise an apparatus arranged to emit a sound when the injection is complete.

In alternative embodiments, the electrical contact apparatus may comprise other types of electrical connecting elements instead of contact pads.

Although claims have been formulated in this application to particular combinations of features, it should be understood that the scope of the disclosure also includes any novel features or any novel combinations of features disclosed herein either explicitly or implicitly or any generalization thereof, whether or not it relates to the same invention as presently claimed in any claim and whether or not it mitigates any or all of the same technical problems as does the present invention. The applicant hereby gives notices that new claims may be formulated to such features and/or combinations of features during the prosecution of the present application or of any further application derived therefrom.

It will be appreciated that the inventive concept of the medical device packaging of the present invention may be applicable to LVDs. However, the invention is not intended to be limited to this particular type of medicament delivery device and the present invention is intended to cover alternative types of medicament delivery devices which include a medicament container to be received in a medicament delivery device which may include, but are not limited to, patch pumps and infusion pumps.

The terms "drug" or "medicament" are used herein to describe one or more pharmaceutically active compounds. As described below, a drug or medicament can include at least one small or large molecule, or combinations thereof, in various types of formulations, for the treatment of one or more diseases. Exemplary pharmaceutically active compounds may include small molecules; polypeptides, peptides and proteins (e.g., hormones, growth factors, antibodies, antibody fragments, and enzymes); carbohydrates and polysaccharides; and nucleic acids, double or single stranded DNA (including naked and cDNA), RNA, antisense nucleic acids such as antisense DNA and RNA, small interfering RNA (siRNA), ribozymes, genes, and oligonucleotides. Nucleic acids may be incorporated into molecular delivery systems such as vectors, plasmids, or liposomes. Mixtures of one or more of these drugs are also contemplated.

The term "drug delivery device" shall encompass any type of device or system configured to dispense a drug into a human or animal body. Without limitation, a drug delivery device may be an injection device (e.g., syringe, pen injector, auto injector, large-volume device, pump, perfusion system, or other device configured for intraocular, subcutaneous, intramuscular, or intravascular delivery), skin patch (e.g., osmotic, chemical, micro-needle), inhaler (e.g., nasal or pulmonary), implantable (e.g., coated stent, capsule), or feeding systems for the gastro-intestinal tract. The presently described drugs may be particularly useful with injection devices that include a needle, e.g., a small gauge needle.

The drug or medicament may be contained in a primary package or "drug container" adapted for use with a drug delivery device. The drug container may be, e.g., a cartridge, syringe, reservoir, or other vessel configured to provide a suitable chamber for storage (e.g., short- or long-term storage) of one or more pharmaceutically active compounds. For example, in some instances, the chamber may be designed to store a drug for at least one day (e.g., 1 to at least 30 days). In some instances, the chamber may be designed to store a drug for about 1 month to about 2 years. Storage may occur at room temperature (e.g., about 20°C), or refrigerated temperatures (e.g., from about - 4°C to about 4°C). In some instances, the drug container may be or may include a dual-chamber cartridge configured to store two or more components of a drug formulation (e.g., a drug and a diluent, or two different types of drugs) separately, one in each chamber. In such instances, the two chambers of the dual-chamber cartridge may be configured to allow mixing between the two or more components of the drug or medicament prior to and/or during dispensing into the human or animal body. For example, the two chambers may be configured such that they are in fluid communication with each other (e.g., by way of a conduit between the two chambers) and allow mixing of the two components when desired by a user prior to dispensing. Alternatively or in addition, the two chambers may be configured to allow mixing as the components are being dispensed into the human or animal body.

The drug delivery devices and drugs described herein can be used for the treatment and/or prophylaxis of many different types of disorders. Exemplary disorders include, e.g., diabetes mellitus or complications associated with diabetes mellitus such as diabetic retinopathy, thromboembolism disorders such as deep vein or pulmonary thromboembolism. Further exemplary disorders are acute coronary syndrome (ACS), angina, myocardial infarction, cancer, macular degeneration, inflammation, hay fever, atherosclerosis and/or rheumatoid arthritis.
Exemplary drugs for the treatment and/or prophylaxis of diabetes mellitus or complications associated with diabetes mellitus include an insulin, e.g., human insulin, or a human insulin analogue or derivative, a glucagon-like peptide (GLP-1), GLP-1 analogues or GLP-1 receptor agonists, or an analogue or derivative thereof, a dipeptidyl peptidase-4 (DPP4) inhibitor, or a pharmaceutically acceptable salt or solvate thereof, or any mixture thereof. As used herein, the term "derivative" refers to any substance which is sufficiently structurally similar to the original substance so as to have substantially similar functionality or activity (e.g., therapeutic effectiveness).

Exemplary insulin analogues are Gly(A21), Arg(B31), Arg(B32) human insulin (insulin glargine); Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Exemplary insulin derivatives are, for example, B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-gamma-glutamyl)-des(B30) human insulin; B29-N-(N-lithocholyl-gamma-glutamyl)-des(B30) human insulin; B29-N-(ω-carboxyheptadecanoyl)-des(B30) human insulin and B29-N-(ω-carboxyhepta¬decanoyl) human insulin. Exemplary GLP-1, GLP-1 analogues and GLP-1 receptor agonists are, for example: Lixisenatide / AVE0010 / ZP10 / Lyxumia, Exenatide / Exendin-4 / Byetta / Bydureon / ITCA 650 / AC-2993 (a 39 amino acid peptide which is produced by the salivary glands of the Gila monster), Liraglutide / Victoza, Semaglutide, Taspoglutide, Syncria / Albiglutide, Dulaglutide, rExendin-4, CJC-1134-PC, PB-1023, TTP-054, Langlenatide / HM-11260C, CM-3, GLP-1 Eligen, ORMD-0901, NN-9924, NN-9926, NN-9927, Nodexen, Viador-GLP-1, CVX-096, ZYOG-1, ZYD-1, GSK-2374697, DA-3091, MAR-701, MAR709, ZP-2929, ZP-3022, TT-401, BHM-034. MOD-6030, CAM-2036, DA-15864, ARI-2651, ARI-2255, Exenatide-XTEN and Glucagon-Xten.

An exemplary oligonucleotide is, for example: mipomersen / Kynamro, a cholesterol-reducing antisense therapeutic for the treatment of familial hypercholesterolemia. Exemplary DPP4 inhibitors are Vildagliptin, Sitagliptin, Denagliptin, Saxagliptin, Berberine.
Exemplary hormones include hypophysis hormones or hypothalamus hormones or regulatory active peptides and their antagonists, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, and Goserelin.
Exemplary polysaccharides include a glucosaminoglycane, a hyaluronic acid, a heparin, a low molecular weight heparin or an ultra-low molecular weight heparin or a derivative thereof, or a sulphated polysaccharide, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium. An example of a hyaluronic acid derivative is Hylan G-F 20 / Synvisc, a sodium hyaluronate.

The term "antibody", as used herein, refers to an immunoglobulin molecule or an antigen-binding portion thereof. Examples of antigen-binding portions of immunoglobulin molecules include F(ab) and F(ab')2 fragments, which retain the ability to bind antigen. The antibody can be polyclonal, monoclonal, recombinant, chimeric, de-immunized or humanized, fully human, non-human, (e.g., murine), or single chain antibody. In some embodiments, the antibody has effector function and can fix complement. In some embodiments, the antibody has reduced or no ability to bind an Fc receptor. For example, the antibody can be an isotype or subtype, an antibody fragment or mutant, which does not support binding to an Fc receptor, e.g., it has a mutagenized or deleted Fc receptor binding region.

The terms "fragment" or "antibody fragment" refer to a polypeptide derived from an antibody polypeptide molecule (e.g., an antibody heavy and/or light chain polypeptide) that does not comprise a full-length antibody polypeptide, but that still comprises at least a portion of a full-length antibody polypeptide that is capable of binding to an antigen. Antibody fragments can comprise a cleaved portion of a full length antibody polypeptide, although the term is not limited to such cleaved fragments. Antibody fragments that are useful in the present invention include, for example, Fab fragments, F(ab')2 fragments, scFv (single-chain Fv) fragments, linear antibodies, monospecific or multispecific antibody fragments such as bispecific, trispecific, and multispecific antibodies (e.g., diabodies, triabodies, tetrabodies), minibodies, chelating recombinant antibodies, tribodies or bibodies, intrabodies, nanobodies, small modular immunopharmaceuticals (SMIP), binding-domain immunoglobulin fusion proteins, camelized antibodies, and VHH containing antibodies. Additional examples of antigen-binding antibody fragments are known in the art.

The terms "Complementarity-determining region" or "CDR" refer to short polypeptide sequences within the variable region of both heavy and light chain polypeptides that are primarily responsible for mediating specific antigen recognition. The term "framework region" refers to amino acid sequences within the variable region of both heavy and light chain polypeptides that are not CDR sequences, and are primarily responsible for maintaining correct positioning of the CDR sequences to permit antigen binding. Although the framework regions themselves typically do not directly participate in antigen binding, as is known in the art, certain residues within the framework regions of certain antibodies can directly participate in antigen binding or can affect the ability of one or more amino acids in CDRs to interact with antigen. Exemplary antibodies are anti PCSK-9 mAb (e.g., Alirocumab), anti IL-6 mAb (e.g., Sarilumab), and anti IL-4 mAb (e.g., Dupilumab).

The compounds described herein may be used in pharmaceutical formulations comprising (a) the compound(s) or pharmaceutically acceptable salts thereof, and (b) a pharmaceutically acceptable carrier. The compounds may also be used in pharmaceutical formulations that include one or more other active pharmaceutical ingredients or in pharmaceutical formulations in which the present compound or a pharmaceutically acceptable salt thereof is the only active ingredient. Accordingly, the pharmaceutical formulations of the present disclosure encompass any formulation made by admixing a compound described herein and a pharmaceutically acceptable carrier.

Pharmaceutically acceptable salts of any drug described herein are also contemplated for use in drug delivery devices. Pharmaceutically acceptable salts are for example acid addition salts and basic salts. Acid addition salts are e.g. HCl or HBr salts. Basic salts are e.g. salts having a cation selected from an alkali or alkaline earth metal, e.g. Na+, or K+, or Ca2+, or an ammonium ion N+(R1)(R2)(R3)(R4), wherein R1 to R4 independently of each other mean: hydrogen, an optionally substituted C1 C6-alkyl group, an optionally substituted C2-C6-alkenyl group, an optionally substituted C6-C10-aryl group, or an optionally substituted C6-C10-heteroaryl group. Further examples of pharmaceutically acceptable salts are known to those of skill in the arts.

Pharmaceutically acceptable solvates are for example hydrates or alkanolates such as methanolates or ethanolates.

Those of skill in the art will understand that modifications (additions and/or removals) of various components of the APIs, substances, formulations, apparatuses, methods, systems and embodiments described herein may be made without departing from the full scope and spirit of the present invention, which encompass such modifications and any and all equivalents thereof.

## Claims

1. A medical device packaging comprising:
a container having an opening;
a medical device disposed within the container, the medical device including an energy storage apparatus;
a first layer comprising a main part disposed at least partially over a peripheral edge of the opening of the container; and
a second layer disposed over the main part of the layer,
wherein the first layer is in electrical contact with the energy storage apparatus and the separation of the lining layer from the conductive layer generates electrostatic energy for charging the energy storage apparatus.

2. The medical device packaging according to claim 1, wherein the main part of the first layer is at least partially adhesive.

3. The medical device packaging according to claim 1 or claim 2, further comprising an electrical contact apparatus, wherein electrostatic energy generated at the first layer is transferred through the electrical contact apparatus to the energy storage apparatus.

4. The medical device packaging according to claim 3, wherein the electrical contact apparatus comprises electrical contact pads arranged to allow electrical connection of the first layer to the energy storage apparatus when the medical device is disposed within the container.

5. The medical device packaging according to claim 3 or claim 4, wherein the first layer comprises an extension part which is arranged to extend towards the electrical contact apparatus such that the first layer is in electrical contact with the electrical contact apparatus.

6. The medical device packaging according to any of the preceding claims, wherein the energy storage apparatus comprises a capacitor.

7. The medical device packaging according to any of the preceding claims, wherein the medical device further comprises a light source arranged to be powered by energy stored in the energy storage apparatus

8. The medical device packaging according to any of the preceding claims, wherein the light source comprises at least one light-emitting diode.

9. The medical device packaging according to any of the preceding claims, wherein the medical device is a bolus injector.

10. The medical device packaging according to any of the preceding claims, wherein the medical device contains liquid medicament.

11. The medical device packaging according to any of the preceding claims, wherein the container is deformable.

12. A method for preparing a medical device packaging for use, the medical device packaging comprising a container having:
an opening;
a medical device disposed within the container, the medical device comprising an energy storage apparatus;
a first layer disposed at least partially over a peripheral edge of the opening of the container, the first layer being in electrical contact with the energy storage apparatus; and
a second layer disposed over the first layer,
the method comprising separating the second layer from the first layer and thereby causing generation of electrostatic energy for charging the energy storage apparatus.
